# EUROPEAN PATENT APPLICATION

(11) **EP 4 632 059 A1**
(43) Date of publication of application: **15.10.2025**
(21) Application number: 23900672.9
(22) Date of filing: 06.12.2023
(51) Int. Cl.: C12N 1/00, A61L 27/36, A61L 27/38, A61L 27/40, C12M 3/00, C12N 5/10

(54) **DECELLULARIZED CELL AGGREGATE AND PRODUCTION METHOD THEREFOR**

(30) Priority: 06.12.2022 JP 2022195029
(71) Applicant: ADEKA CORPORATION, Arakawa-ku Tokyo 116-8554 (JP)
(72) Inventor: MUTO, Mio, Tokyo 116-8554 (JP); TOSAKA, Hotaru, Tokyo 116-8554 (JP); SATO, Moe, Tokyo 116-8554 (JP); HIWATARI, Kenichiro, Tokyo 116-8554 (JP); KAWAHARA, Nana, Tokyo 116-8554 (JP); OBARA, Mai, Tokyo 116-8554 (JP)
(74) Representative: Gille Hrabal Partnerschaftsgesellschaft mbB Patentanwälte
(86) International application number: PCT/JP2023/043572
(87) International publication number: WO 2024/122558

(57) **Abstract**

The object of the present invention is to provide a decellularized graft which is easily manufactured.

The problem can be solved by a decellularized cell aggregate, wherein a cultured cell aggregate is decellularized, and surface pores with pore diameter of 5µm or more are 3 to 45 per 11088µm² of aggregate surface, and/or a decellularized cell aggregate, wherein a cultured cell spheroid is decellularized, and lamin of 10pg/mg or more is contained with respect to the decellularized cell aggregate.

## Description

### TECHNICAL FIELD

The present invention relates to a decellularized cell aggregate and a method for preparing the same.

### BACKGROUND ART

When transplanting a graft derived from a living tissue of another person or another species of animal, graft rejection by the recipient tissue is problematic. In order to improve the compatibility of grafts with biological tissues, techniques have been developed to use decellularized tissues consisting of the remaining supportive tissues (extracellular matrix, ECM) after removal of cells from biological tissues as grafts. Decellularization means the removal of cellular components such as nucleic acids that are antigenic to the recipient, thereby avoiding immune rejection (Patent literatures 1 and 2).

### CITATION LIST

### PATENT LITERATURE

[Patent literature 1] JP 2005-514971 A
[Patent literature 2] WO 2016/136633

### SUMMARY OF INVENTION

### TECHNICAL PROBLEM

The decellularized tissues are useful in that decellularization of living tissues yields grafts that are free from rejection. However, it is necessary to use animal-derived biological tissue and is difficult to manufacture, and thus a more easily manufactured graft is desired.

Therefore, the object of the present invention is to provide a decellularized graft which is easily manufactured.

### SOLUTION TO PROBLEM

The present inventors have conducted intensive studies into a decellularized graft which is easily manufactured., as a result, surprisingly found that the graft can be easily obtained by decellularizing cell aggregate.

The present invention is based on the above findings.

Therefore, the present invention relates to:
[1] a decellularized cell aggregate, wherein a cultured cell aggregate is decellularized, and surface pores with pore diameter of 5µm or more are 3 to 45 per 11088µm² of aggregate surface,
[2] a decellularized cell aggregate, wherein a cultured cell aggregate is decellularized, and lamin of 10pg/mg or more is contained with respect to the decellularized cell aggregate,
[3] a decellularized cell aggregate, wherein a cultured cell aggregate is decellularized, surface pores with pore diameter of 5µm or more are 3 to 45 per 11088µm² of aggregate surface, and lamin of 10pg/mg or more is contained with respect to the decellularized cell aggregate,
[4] the decellularized cell aggregate of any one of the items [1] to [3], wherein cells of the cultured cell aggregate are immortalized cells,
[5] the decellularized cell aggregate of any one of the items [1] to [3], wherein cells of the cultured cell aggregate are a mixture of two or more kinds of cells,
[6] the decellularized cell aggregate of any one of the items [1] to [3], wherein a DNA content in dry mass is 1.000 % by weight or less with respect to the decellularized cell aggregate,
[7] a cell culture substrate comprising the decellularized cell aggregate of any one of the items [1] to [3],
[8] a graft for living body comprising the decellularized cell aggregate of any one of the items [1] to [3],
[9] a composition comprising the decellularized cell aggregate of any one of the items [1] to [3], and cells,
[10] a method for preparing a decellularized cell aggregate comprising a formation step of cultured cell aggregate wherein the cultured cell aggregate is formed by culturing cells, and a decellularization step wherein the cultured cell aggregate is decellularized, wherein surface pores with pore diameter of 5µm or more are 3 to 45 per 11088µm² of aggregate surface
[11] a method for preparing a decellularized cell aggregate comprising a formation step of cultured cell aggregate wherein the cultured cell aggregate is formed by culturing cells, and a decellularization step wherein the cultured cell aggregate is decellularized, wherein lamin of 10pg/mg or more is contained with respect to the decellularized cell aggregate
[12] the method for preparing a decellularized cell aggregate of the item [10] or [11], wherein the cells are immortalized cells,
[13] the method for preparing a decellularized cell aggregate of the item [10] or [11], wherein the culture of cells is a culture of two or more kinds of cells
[14] The method for preparing a decellularized cell aggregate of the item [10] or [11], wherein the decellularization is performed by high hydrostatic pressure treatment.

### ADVANTAGEOUS EFFECTS OF INVENTION

According to the method for preparing decellularized cell aggregate of the present invention, the graft can be easily obtained. Further, the decellularized cell aggregate of the present invention has specific physical properties, and thus exhibits an excellent cell viability as the scaffold of cells.

### DESCRIPTION OF EMBODIMENTS

[Fig. 1] Fig. 1 is a graph showing the area maintenance rates of Examples 1 to 8 and Comparative Example 1 according to the method for preparing decellularized cell aggregate of the present invention.
[Fig. 2] Fig. 2 is photographs of SEM showing the conditions of surfaces of decellularized cell aggregates of Examples 1 to 8 and Comparative Examples 1 and 2.
[Fig. 3] Fig. 3 is a graph showing the results of measured surface pores of decellularized cell aggregates of Examples 1 to 8 and Comparative Examples 1 and 2.
[Fig. 4] Fig. 4 is a graph showing the measured cell viabilities in the cell cultures using decellularized cell aggregates of Examples 1 to 8 and Comparative Examples 1 and 2.
[Fig. 5] Fig. 5 is photographs of decellularized cell aggregates of Examples 1 to 8 and Comparative Example 1 stained by immunohistochemistry using an antibody against lamin B1.
[Fig. 6] Fig. 6 is a graph showing quantified lamin B1 in the decellularized cell aggregates of Examples 1 to 6 and 8 measured by enzyme immunoassay.
[Fig. 7] Fig. 7 is a graph showing the calculated lamin B1-positive areas of Examples 1 to 8 by importing images of the immunohistochemically stained sections and using image processing software (ImageJ).

### DESCRIPTION OF EMBODIMENTS

### [1] Decellularized cell aggregate

One embodiment of the decellularized cell aggregate of the present invention is a decellularized cell aggregate, wherein a cultured cell aggregate is decellularized, and surface pores with pore diameter of 5µm or more are 3 to 45 per 11088µm² of aggregate surface.

One embodiment of the decellularized cell aggregate of the present invention is a decellularized cell aggregate, wherein a cultured cell aggregate is decellularized, and lamin of 10pg/mg or more is contained.

The decellularized cell aggregate of the present invention may have surface pores of 3 to 45 with pore diameter of 5µm or more per 11088µm² of aggregate surface and may contain lamin of 10pg/mg or more.

It is preferable that the cells of the cultured cell aggregate are immortalized cells. It is presumed that by preparing the decellularized cell aggregate using immortalized cells, it is possible that a large amount of lamin described later is contained, and the area maintenance rate and the number of surface pores are adjusted to a predetermined value. This allows the cell viability to be increased when cells are seeded on the decellularized cell aggregate.

It is preferable that the cells of the cultured cell aggregate are a mixture of two or more types of cells. It is presumed that by preparing the decellularized cell aggregate by co-culturing cells, it is possible that a large amount of lamin described later is contained, and the area maintenance rate and the number of surface pores are adjusted to a predetermined value. This allows the cell viability to be increased when cells are seeded on the decellularized cell aggregate.

### <<Cultured cell aggregate>>

The cultured cell aggregate is not particularly limited, as long as it is a cell aggregate obtained by culturing in vitro. Specifically, cells produce extracellular matrix (ECM) outside the cells by three-dimensional culture, and whereby the cells to adhere and maintain the shape of a three-dimensional aggregate, and there may be mentioned spheroid. Furthermore, the cultured cell aggregate may be a cell block in which multiple spheroids are adhered together.

The shape of the cultured cell aggregate is not particularly limited, and there may be mentioned a sphere, an elliptical sphere, a cube, a rectangular parallelepiped, a cylinder, and a square prism. When the cultured cell aggregate is a spheroid, the shape is often the cylinder or the elliptical sphere. The size of the cultured cell aggregate is not particularly limited, but in the case of the spheroid, the shortest length is 80 µm or more, preferably 90 µm or more, more preferably 95 µm or more, and even more preferably 100 µm or more. The longest length is also not limited, but is 600 µm or less, preferably 500 µm or less, and more preferably 300 µm or less.
In the case of the cell block, the shortest length is 90 µm or more, preferably 100 µm or more, more preferably 150 µm or more, and even more preferably 200 µm or more. The longest length is also not limited, but is 2100 µm or less, preferably 2000 µm or less, more preferably 1700 µm or less, even more preferably 1400 µm or less, even more preferably 1200 µm or less, and most preferably 1000 µm. The upper and lower limits can be combined to form a range of the size of the cultured cell aggregate. This allows the effects of the present invention to be exhibited satisfactorily.

The spheroids can be prepared by three-dimensionally culturing cells and aggregating them into a spherical shape or the like. Spheroids are formed from cells and extracellular matrix (ECM), and contain more extracellular matrix than cell populations prepared by two-dimensional culture (monolayer culture).

Methods for three-dimensionally culturing spheroids include, but are not limited to, a non-adhesive surface culture method, a hanging drop culture method, or a rotary culture method.

In the non-adhesive surface culture method, spheroids are obtained using a non-adhesive culture plate with improved hydrophilicity on the plate surface. The cells contained in the non-adhesive culture plate float in the medium and form spheroids by adhering to each other. By enlarging the culture plate, a large number of spheroids can be cultured.

In the hanging drop culture method, suspended cells are dropped onto the inside of the lid of a culture plate, and the droplets that rise due to surface tension are cultured. The cells, which gather at the bottom due to gravity, adhere to each other, and form spheroids. In the hanging drop culture method, it is relatively easy to keep the number of cells in one spheroid constant.

In the rotary culture method, spheroids are formed by rotating the culture chamber. The rotation allows the suspended cells to come into contact with each other evenly, and it possible to prepare spheroids with a relatively uniform cell number and size. In addition, the rotary culture method also allows for large-scale culture.

The cells used for the cultured cell aggregate are broadly classified into suspension cells and adherent cells. The cells for the cultured cell aggregate are not limited, but from the viewpoint of ease of preparation of the cultured cell aggregate, adherent cells are preferrable.

The adherent cells are not particularly limited and can be appropriately selected depending on the purpose of use of the cultured cell aggregate. For example, differentiated adherent cells include hepatocytes, astrocytes, Kupffer cells, vascular endothelial cells, sinusoidal endothelial cell, endothelial cells, osteoblasts, osteoclasts, periodontal ligament derived cells, epidermal cells, tracheal epithelial cells, gastrointestinal epithelial cells, cervical epithelial cells, epithelial cells, mammary gland cells, pericytes, smooth muscle cells, cardiac muscle cells, myocytes, renal cells, pancreatic islet cells of Langerhans, peripheral nerve cells, neurons, chondrocytes, or osteocytes. For example, adherent cells capable of differentiation include embryonic stem cells (ES cells), embryonic germ cells (EG cells), germline stem cells (GS cells), induced pluripotent stem cells (iPS cells), mesenchymal stem cells, hematopoietic stem cells, neural stem cells, cardiac progenitor cells, vascular endothelial progenitor cells, neural progenitor cells, adipose progenitor cells, fibroblasts, dermal fibroblasts, skeletal muscle myoblasts, osteoblasts, or odontoblasts. Two or more types of cells may be used. From the viewpoint of ease of preparing cultured cell aggregate, fibroblasts, endothelial cells, mesenchymal stem cells, and cardiac progenitor cells are preferrable, and dermal fibroblasts, vascular endothelial cells, mesenchymal stem cells, and cardiac progenitor cells are more preferrable.

In addition, immortalized cells can be used as the cells used in the cultured cell aggregates. Immortalized cells refer to cells whose proliferation does not cease even after repeated cell division. Immortalized cells may be produced (immortalized) by artificially introducing a gene into the above-mentioned cells. From the viewpoint of exerting the effects of the present invention, it is preferable that the immortalized cells are immortalized by artificially introducing a gene into the above-mentioned cells.

Immortalized cells are not particularly limited, but include myeloma cell lines, immortalized fibroblasts, and immortalized vascular endothelial cells. Immortalized fibroblasts and immortalized vascular endothelial cells are preferable from the viewpoint of exerting the effects of the present invention. Myeloma cell lines include, for example, NSI-Ag4/1 and Sp2/O-Agl4. Immortalized fibroblasts include, for example, OUMS-36T-1 and TelCOFS02MA, and OUMS-36T-1 is preferable from the viewpoint of exerting the effects of the present invention. The immortalized vascular endothelial cell includes HUEhT-1.

As the cells to be used for the cultured cell aggregate, from the viewpoint of increasing cell viability when a decellularized cell aggregate is prepared by, for example, the method for preparing decellularized cell aggregate described below and cells are seeded into the decellularized cell aggregate, it is preferable to use immortalized cells, more preferably immortalized fibroblasts or immortalized endothelial cells, and even more preferably immortalized fibroblasts.

An origin of cell is not particularly limited in origin, and the cells include eukaryotic cells, prokaryotic cells, multicellular organism cells, or unicellular organism cells. Eukaryotic cells include animal cells, insect cells, plant cells, fungi, algae, or protozoa. The animal cell is not limited, but includes cells derived from human, monkey, dog, cat, rabbit, ferret, sheep, goat, cow, pig, horse, camel, mouse, rat, hamster, guinea pig, or gerbil.

The spheroids are preferably produced by preparing decellularized cell aggregates by co-culturing different types of cells from the viewpoint of increasing cell viability when a decellularized cell aggregate is prepared by, for example, the method for preparing decellularized cell aggregate described below and cells are seeded into the decellularized cell aggregate. The co-culture of cells in the preparation of the spheroids can use two or more types of cells. From the viewpoint of further exerting the effects of the present invention, it is preferable to use three types of cells, and it is more preferable to use two types of cells. The cells used in the co-culture of cells in the production of the spheroids are not particularly limited, but can be selected from the cells used in the above-mentioned cultured cell aggregate, and from the viewpoint of further exerting the effects of the present invention, it is preferable to select from fibroblasts and endothelial cells, and it is more preferable to use dermal fibroblasts and vascular endothelial cells. The cells used in the co-culture of cells in the preparation of the spheroid may be immortalized, and from the viewpoint of further exerting the effects of the present invention, the dermal fibroblasts and immortalized vascular endothelial cells are most preferable.

The cultured cell aggregate is preferably, but not limited to, adhered by an extracellular matrix. Adhesion by an extracellular matrix allows the cultured cell aggregate to obtain a certain strength. The extracellular matrix includes collagen, laminin, fibronectin, chondroitin sulfate, heparin sulfate, keratan sulfate, and hyaluronic acid, but they varies depending on the type of cell and is not particularly limited.

The decellularized cell aggregate of the present invention can be obtained by decellularizing the cultured cell aggregate, for example, using the method for preparing decellularized cell aggregate described below, without being limited thereto. In particular, it is possible to obtain a decellularized cell aggregate having uniform physical properties by decellularizing the spheroid. The decellularized cell aggregate of the present invention may be in a wet state or a dry state. The decellularized cell aggregate provided in a wet state can be used immediately for transplantation. On the other hand, the decellularized cell aggregate provided in a dry state can be stored for a long period of time, can be easy to transport, and can be restored and used when in use.

### <<Surface pore>>

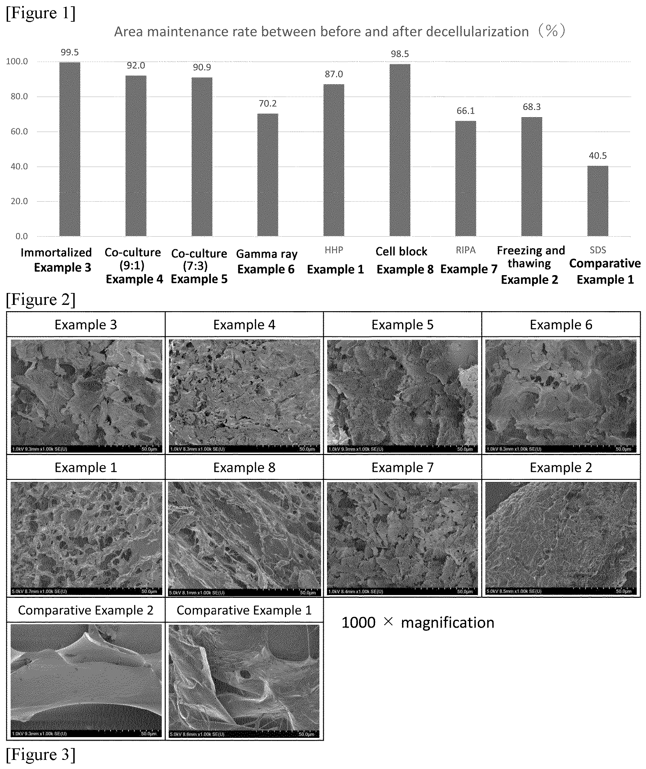

The decellularized cell aggregate of the present invention has 3 to 45 of surface pores with pore diameter of 5 µm or more per 11,088 µm² of the aggregate surface. In the present specification, surface pores mean pores present on the surface of the decellularized cell aggregate when the decellularized cell aggregate is observed with a scanning electron microscope (SEM). Furthermore, the surface refers to the surface that can be observed with the SEM. As shown in Figure 2, the decellularized cell aggregate of the present invention has a specific number of surface pores with a diameter of 5 µm or more. By having a certain number of surface pores, it exhibits excellent effects as a scaffold for cell culture.

The diameter of the surface pores to be counted is not particularly limited as long as it is 5 µm or more, but the lower limit is preferably 5 µm or more and the upper limit is preferably 50 µm or less. The diameter of the surface pores can be mechanically or visually measured from the SEM photograph.

The number of surface pores per 11088 µm² of the cultured cell aggregate surface is not particularly limited as long as it is 3 to 45, but the lower limit is preferably 3 or more in one embodiment, 4 or more in one embodiment, 5 or more in one embodiment, 6 or more in one embodiment, 7 or more in one embodiment, 8 or more in one embodiment, 9 or more in one embodiment, 10 or more in one embodiment, 11 or more in one embodiment, 12 or more in one embodiment, 13 or more in one embodiment, 14 or more in one embodiment, and 15 or more in one embodiment. The upper limit of the number of surface pores is 45 or less, preferably 43 or less in one embodiment, 41 or less in one embodiment, 39 or less in one embodiment, 37 or less in one embodiment, 35 or less in one embodiment, 33 or less in one embodiment, 31 or less in one embodiment, 30 or less in one embodiment, 28 or less in one embodiment, 26 or less in one embodiment, 24 or less in one embodiment, 22 or less in another embodiment, 20 or less in one embodiment, 18 or less in one embodiment. The upper and lower limits can be combined to form a range for the number of surface pores. The survival number of cells can be increased by keeping the number within the above range.

The method for identifying the aggregate surface area (11088 µm²) for measuring surface pores is not particularly limited, but for example, the number of surface pores in an area of 88 µm × 126 µm on an SEM photograph may be counted.

### <<Lamin>>

The decellularized cell aggregate of the present invention contain 10 pg/mg or more of lamin with respect to the decellularized cell aggregate. Lamins include lamin A, lamin B1, lamin B2, and lamin C. The lamin contained in the decellularized cell aggregate of the present invention is not limited, but preferably lamin B1 (SEQ ID NO: 1). However, the decellularized cell aggregate of the present invention may contain lamin A, lamin B2, and lamin C.

Lamin B1 is encoded by the LMNB1 (also known as LMN2 or LMNB) gene located at 5q23 in humans, and is a homodimeric protein synthesized with a short propeptide (aa584-586). Lamin is a fibrous protein that maintains structure and regulates transcription in the cell nucleus, and forms the nuclear lamina on the inside of the nuclear membrane together with membrane proteins. The nuclear lamina controls the cell cycle, and the disassembly and formation of the nuclear membrane. During mitosis (somatic cell division), lamin proteins are phosphorylated and The nuclear lamina is reversibly disassembled.

The content of lamin in the decellularized cell aggregate is not particularly limited as long as it is 10 pg/mg or more, but the lower limit is preferably 15 pg/mg or more in one embodiment, 18 pg/mg or more in one embodiment, 20 pg/mg or more in one embodiment, 25 pg/mg or more in one embodiment, 30 pg/mg or more in one embodiment, 35 pg/mg or more in one embodiment, 40 pg/mg or more in one embodiment, 45 pg/mg or more in one embodiment, 50 pg/mg or more in one embodiment, 60 pg/mg or more in one embodiment, 80 pg/mg or more in one embodiment, 100 pg/mg or more in one embodiment, 110 pg/mg or more in one embodiment, 120 pg/mg or more in one embodiment, 130 pg/mg or more in one embodiment, and 135 pg/mg or more in one embodiment. The upper limit is also not particularly limited, but it is preferably 5000pg/mg or less in one embodiment, 4000pg/mg or less in one embodiment, 3000pg/mg or less in one embodiment, 2000pg/mg or less in one embodiment, 1000pg/mg or less in one embodiment, 900pg/mg or less in one embodiment, 800pg/mg or less in one embodiment, 700pg/mg or less in one embodiment, 600pg/mg or less in one embodiment, 500pg/mg or less in one embodiment, 450pg/mg or less in one embodiment, 400pg/mg or less in one embodiment, 350pg/mg or less in one embodiment, 300pg/mg or less in one embodiment, 250pg/mg or less in one embodiment, 200pg/mg or less in one embodiment, 150pg/mg or less in one embodiment. The upper and lower limits can be appropriately combined to form a range for the lamin content.

An excellent cell viability is exhibited by keeping the lamin content within the above range.

In the present specification, detection and quantification of lamin can be performed, for example, as follows. An section of decellularized cell aggregate is stained by immunohistochemical staining in which lamin can be stained, and lamin is detected. Then, the lamin content of the decellularized cell aggregate is quantified by enzyme-linked immunosorbent assay (ELISA).

For example, Immunohistochemical staining can be performed as follows. A section sample of decellularized cell aggregate is treated with primary and secondary antibodies. Subsequently, the samples are colored with DAB (3,3'-diaminobenzidine tetrahydrochloride)/hydrogen peroxide, dehydrated, and mounted.

For example, enzyme-linked immunosorbent assay can be performed as follows. Using an extract of the decellularized cell aggregate, the lamin content of the decellularized cell aggregate is quantified by ELISA.

### <<DNA content>>

The DNA content per dry mass of the decellularized cell aggregate is not particularly limited, but is 1.000% by mass or less.
The DNA content per dry mass of the decellularized cell aggregate is 0.900% by mass or less, preferably 0.800% by mass or less, more preferably 0.700% by mass or less, even more preferably 0.600% by mass or less, even more preferably 0.500% by mass or less, even more preferably 0.400% by mass or less, even more preferably 0.300% by mass or less, even more preferably 0.250% by mass or less, and whereby it is possible to obtain a decellularized cell aggregate which is properly decellularized and has less rejection when transplanted.

The DNA content can be measured by the PicoGreen method. A dried specimen of the decellularized cell aggregate (hereinafter referred to as "sample") is immersed in proteolytic enzyme solution to dissolve the same. Then, it is treated with phenol/chloroform to remove proteins, and DNA is recovered. Treatment with phenol/chloroform to remove proteins may be followed by ethanol precipitation. The recovered DNA is fluorescently stained with PicoGreen (Life Technologies) and the DNA is quantified by measuring the fluorescence intensity, and the DNA content (mass) of the sample is calculated. For quantification, a calibration curve prepared using the standard DNA provided with Picogreen is used. From the dry mass and DNA content of the sample, the DNA ratio is calculated according to the following formula. (DNA content per dry mass (hereinafter sometimes referred to as decellularized DNA ratio)) = (DNA content of dried specimen of decellularized cell aggregate)/(mass of dried specimen of decellularized cell aggregate)

### <<Cell culture substrate>>

The decellularized cell aggregate of the present invention can be used as a substrate for cell culture. As a culture method, any known method can be used, except for using the decellularized cell aggregate of the present invention.
For example, a predetermined amount of medium or culture reagent can be placed in a cell culture plate or cell culture well, the decellularized cell aggregate of the present invention can be added, cells can be seeded, and cultured under a predetermined temperature and CO₂ concentration conditions. The medium or culture reagent can be appropriately selected depending on the cells to be cultured.

The cells to be cultured are not particularly limited, for example, and there may be mentioned the same cells as those used in the above-mentioned "Cultured cell aggregate."

### (Cell viability)

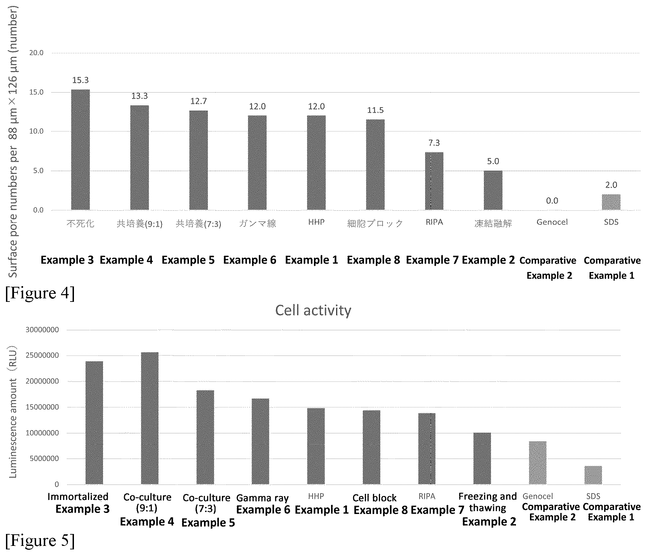

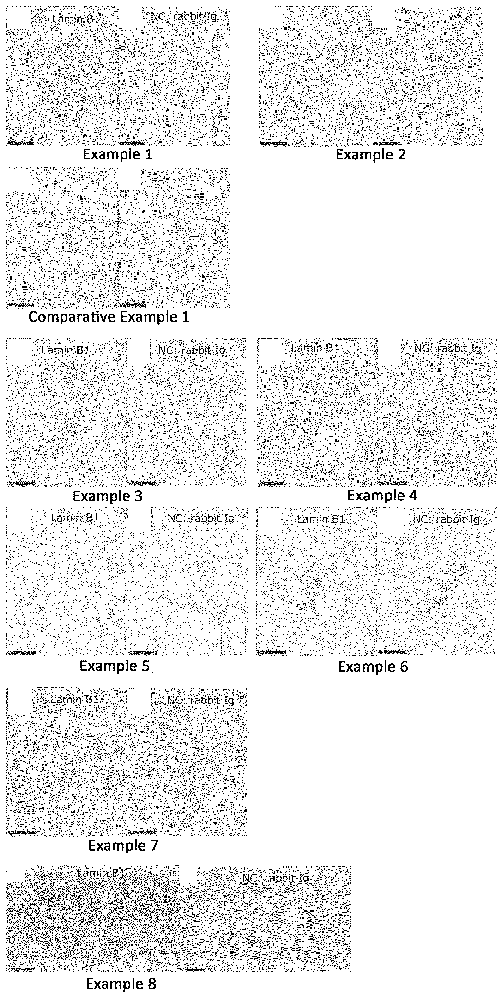

When the decellularized cell aggregate of the present invention is used as the cell culture substrate, it exhibits excellent cell viability (Figure 4). Cell viability can be analyzed by counting the number of cells after culturing the cells with the decellularized cell aggregate, but it can also be analyzed by measuring ATP as shown in the Examples.

### <<Graft>>

The decellularized cell aggregate of the present invention can be used as a graft for a living body. The decellularized cell aggregate of the present invention has excellent biocompatibility, and it can serve, for example, as a suitable scaffold for cell growth, by transplanting into a living body.

Living organisms into which the decellularized cell aggregate of the present invention may be transplanted include human, monkey, dog, cat, rabbit, ferret, sheep, goat, cow, pig, horse, camel, mouse, rat, hamster, guinea pig, or gerbil.

Diseases for which the graft of the present invention can be transplanted include cell-decreasing diseases (cell-loss diseases). Cells that are decreased (lost) in the body include hepatocytes, astrocytes, Kupffer cells, vascular endothelial cells, sinusoidal endothelial cell, endothelial cells, fibroblasts, osteoblasts, osteoclasts, periodontal ligament derived cells, epidermal cells, tracheal epithelial cells, gastrointestinal epithelial cells, cervical epithelial cells, epithelial cells, mammary gland cells, pericytes, smooth muscle cells, cardiac muscle cells, myocytes, renal cells, pancreatic islet cells of Langerhans, peripheral nerve cells, neurons, chondrocytes, or osteocytes. For example, undifferentiated adherent cells include embryonic stem cells (ES cells), embryonic germ cells (EG cells), germline stem cells (GS cells), induced pluripotent stem cells (iPS cells), mesenchymal stem cells, hematopoietic stem cells, neural stem cells, cardiac progenitor cells, vascular endothelial progenitor cells, neural progenitor cells, adipose progenitor cells, dermal fibroblasts, skeletal muscle myoblasts, osteoblasts, or odontoblasts. For these diseases in which cells are decreased, transplantation of the graft of the present invention can effectively increase the number of cells. The cells may also be transplanted into the body at the same time as transplantation of the graft of the present invention.

Furthermore, the graft of the present invention can also be transplanted in vivo into an individual in which the cells are not decreased. Various cells can be proliferated by administration of the graft.

### <<Composition>>

The composition of the present invention comprises decellularized cell aggregate and cells. The cells include hepatocytes, astrocytes, Kupffer cells, vascular endothelial cells, sinusoidal endothelial cell, endothelial cells, fibroblasts, osteoblasts, osteoclasts, periodontal ligament derived cells, epidermal cells, tracheal epithelial cells, gastrointestinal epithelial cells, cervical epithelial cells, epithelial cells, mammary gland cells, pericytes, smooth muscle cells, cardiac muscle cells, myocytes, renal cells, pancreatic islet cells of Langerhans, peripheral nerve cells, neurons, chondrocytes, or osteocytes. For example, undifferentiated adherent cells include embryonic stem cells (ES cells), embryonic germ cells (EG cells), germline stem cells (GS cells), induced pluripotent stem cells (iPS cells), mesenchymal stem cells, hematopoietic stem cells, neural stem cells, cardiac progenitor cells, vascular endothelial progenitor cells, neural progenitor cells, adipose progenitor cells, dermal fibroblasts, skeletal muscle myoblasts, osteoblasts, or odontoblasts. Two or more types of cells may be used. As the above cells, immortalized cells can be used, as described in the section on cells used for the cultured cell aggregate.

The ratio of cells to decellularized cell aggregate is not particularly limited as long as it is equal to or less than the number of cells that can be physically contained in the decellularized cell aggregate. For example, 1.0×10³ to 1.0×10⁷ cells can be contained per 1 mg of decellularized cell aggregate. The lower limit of the number of cells is 2.0×10³ or more in one embodiment, 5.0×10³ or more in one embodiment, 1.0×10⁴ or more in one embodiment, 2.0×10⁴ or more in one embodiment, and 5.0×10⁴ or more in one embodiment. The upper limit of the number of cells is 5.0×10⁶ or less, 1.0×10⁶ or less in one embodiment, 5.0×10⁵ or less in one embodiment, 2.0×10⁵ or less in one embodiment, and 1.0×10⁵ or less in one embodiment.
The upper and lower limits can be arbitrarily combined to form a range for of the ratio of cells to decellularized cell aggregate. The survival number of cells can be increased by keeping the ratio within the above range.

The composition of the present invention is not particularly limited, so long as it contains decellularized cell aggregate and cells, and can be used for in vitro cell culture. It can also be administered to a living body and used, for example, for autologous or allogeneic transplantation in regenerative medicine or cell therapy.

### [2] Method for preparing decellularized cell aggregate

One embodiment of the method for preparing a decellularized cell aggregate of the present invention comprises a formation step of cultured cell aggregate wherein the cultured cell aggregate is formed by culturing cells, and a decellularization step wherein the cultured cell aggregate is decellularized, and wherein surface pores with pore diameter of 5µm or more are 3 to 45 per 11088µm² of aggregate surface.

One embodiment of the method for producing a decellularized cell aggregate of the present invention comprises a formation step of cultured cell aggregate wherein the cultured cell aggregate is formed by culturing cells, and a decellularization step wherein the cultured cellular aggregate is decellularized, and wherein lamin of 10pg/mg or more is contained with respect to the decellularized cell aggregate

The decellularized cellular aggregate obtained by the method for preparing a decellularized cell aggregate of the present invention may have 3 to 45 surface pores with a pore diameter of 5 µm or more per 11088µm² of aggregate surface, and contain 10 pg/mg or more of lamin with respect to the decellularized cell aggregate.

### <<Formation step of cultured cell aggregate (1)>>

In the Formation step of cultured cell aggregate (1), cells are cultured to form cultured cell aggregate. The cultured cell aggregate is not particularly limited as long as it is a cell aggregate obtained by in vitro culture, but include the "cultured cell aggregate " described in the above section "[1] Decellularized cell aggregate ". The cultured cell aggregate can be formed when cells produce extracellular matrix (ECM) outside the cells, and adhere and bond cells to each other.

In the case of spheroids, which are one type of cultured cell aggregate, three-dimensional culture methods include, for example, the non-adhesive surface culture method, the hanging drop culture method, and the rotary culture method. The spheroid formation in the formation step of cultured cell aggregate (1) is specifically described below.

The cultured cell aggregates (such as spheroids) can be prepared as follows. Adherent cells cannot survive for a long period of time when they are floating in solution. When the adherent cells are placed in a non-adherent environment, they adhere to each other in search of scaffolds and form cell aggregates.

Specifically, the cultured cell aggregate (such as spheroids) can be prepared by aggregating multiple cells. After cells are cultured in a monolayer, they are transferred to water-repellent or non-adherent round-bottomed multi-wells or U-shaped plate (dimple plate) and incubated, and whereby cells are aggregated to form cultured cell aggregates. The medium used for cell culture to prepare cultured cell aggregate (such as spheroid) can be selected according to the cells used. From the viewpoint of efficiency, an incubation time to form cultured cell aggregates is preferably 6 to 48 hours, more preferably 6 to 24 hours. However, the method for preparing cultured cell aggregates is not limited to the above methods, and the gyratory culture method in which cell suspensions are placed in a gyrating solution, the method in which cell suspensions are placed in test tubes and precipitated by a centrifuge, or the arginate bead method, can be used. From the viewpoint that a large amount of uniform cell aggregates can be processed, the method of placing cell suspensions in water-repellent or cell-nonadherent multi-wells is efficient and preferrable.

By adjusting the incubation time of cultured cell aggregates, the cultured cell aggregate can be cultured to any desired thickness. In addition, by adhering cultured cell aggregates (such as spheroids) together, the cultured cell aggregates can be cultured to any thickness and shape. By further culturing the obtained cultured cell aggregates, the cells secrete extracellular matrix, the cells adhere to each other, and structural cultured cell aggregates maintaining a three-dimensional shape can be obtained. In addition, by culturing the cultured cell aggregates (such as spheroids) in a cavity as described below, multiple spheroids are bonded to precisely prepare cultured cell aggregate which is cell blocks with a desired three-dimensional shape. Furthermore, the cell blocks can be prepared using the method for producing sheet-like 3D structure, the method for stacking multiple cell sheets, or the like.

The medium used for cell culture to prepare cultured cell aggregates (such as cell blocks) can be selected according to the type of cells used. The cultured cell aggregate is placed in a cavity surrounded by reticular or comb-like supports with gaps smaller than the size of the cultured cell aggregate. The cavity is formed into a desired shape by the reticular or comb-like supports and plates, and the supports have many openings. The medium can be brought into contact with the cultured cell aggregates through these openings. While maintaining the cultured cell aggregates in the cavity, a container for shaking culture is shaken, and whereby the medium is effectively brought in to contact with the cultured cell aggregates and the cultured cell aggregates are cultured well. The cultured cell aggregates further produce the extracellular matrix, and the cell aggregates begin to adhere to each other, and the cell block with a desired three-dimensional shape can be produced.

The cells used to prepare the cultured cell aggregate are not particularly limited, but the cells described in the above item "[1] Decellularized cell aggregate " can be used.

The cells in the formation step of cultured cell aggregate are not limited, but are preferably immortalized cells. It is presumed that when decellularized cell aggregates are prepared by the method for preparing decellularized cell aggregate, the area maintenance rate and the number of surface pores can be effectively set to predetermined values by containing a large amount of lamin. This allows the cell viability to be increased when cells are seeded into the decellularized cell aggregates

In another embodiment, it is preferable that the cell culture in the formation step of cultured cell aggregate is the culture of two or more types of cells. It is presumed that by producing a decellularized cell aggregate using the method for preparing a decellularized cell aggregate in which cells are co-cultured, a large amount of lamin are contained therein, and the area maintenance rate and the number of surface pores can be effectively set to a predetermined value. This makes it possible to increase cell viability when cells are seeded into the decellularized cell aggregate.

The procedure for preparing cultured cell aggregates (such as spheroids) by co-culture is as follows: the same type of cells is cultured in a monolayer and then they are detached, and different types of cells are mixed to a predetermined cell number ratio, to prepare a suspension. Thereafter, similar to the preparation of the above cultured cell aggregate (such as spheroids), the cells can be transferred to a water-repellent or non-adherent round-bottom multi-well or U-shaped plate (dimple plate) and incubated, i.e., co-cultured. The cells may or may not have formed spheroids at the start of co-culture, but from the viewpoint of exerting the effects of the present invention, it is preferable that the cells have not formed spheroids at the start of co-culture. For the co-culture of cells in the preparation of spheroids, two or more types of cells can be used, and from the viewpoint of exerting the effects of the present invention, it is preferable to use three types of cells, and more preferable to use two types of cells. For example, when two types of cells are used, the mixing ratio of the two types of cells is 1:99 to 99:1, it is 10:90 to 90: 10 in one embodiment, 20:80 to 80:20 in one embodiment, 30:70 to 70:30 in one embodiment, 40:60 to 60:40 in one embodiment, 50:50 in one embodiment.

The cells used in the co-culture of cells in the preparation of the spheroids are not particularly limited, but can be selected from the cells used in the cultured cell aggregates described above. From the viewpoint of exerting the effects of the present invention more effectively, the cells are preferably selected from fibroblasts and endothelial cells, and more preferably dermal fibroblasts and vascular endothelial cells. The cells used in the co-culture of cells in the preparation of the spheroids may be immortalized, and from the viewpoint of exerting the effects of the present invention more effectively, the cells are most preferably dermal fibroblasts and immortalized vascular endothelial cells. The ratio of dermal fibroblasts to vascular endothelial cells is not particularly limited as long as the effects of the present invention can be obtained, but is, for example, 2:8 to 8:2, 3:7 to 7:3 in one embodiment, 4:6 to 6:4 in another embodiment.
In addition, there may be an embodiment in which the number of fibroblasts is greater than the number of vascular endothelial cells, but the ratio of dermal fibroblasts to vascular endothelial cells is preferably 2:1 to 15:1, more preferably 4:1 to 12:1, and more preferably 6:1 to 10:1. The upper and lower limits can be appropriately combined. Although not limiting, the effect of increasing cell viability when cells are seeded on the decellularized cell aggregate of the present invention can be more effectively achieved, by keeping the ratio within the above range.

### <<Decellularization step (2)>>

In the decellularization step (2), the cultured cell aggregate is decellularized. The decellularization treatment is not particularly limited as long as the effects of the present invention can be obtained, but there may be mentioned a high hydrostatic pressure treatment, freezing and thawing method, ultrasonic treatment, enzyme treatment, treatment with hypertonic electrolyte solution, a physical agitation, hypertonic solution/hypotonic solution method, enzyme treatment with proteolytic enzymes or nucleolytic enzyme, and treatment with an alcohol solvent, or the like, and a combination of two or more of these methods may be used. In order to efficiently obtain the decellularized cell aggregate and to achieve the effect of the present invention, the method of high hydrostatic pressure treatment is preferable.

### <<High hydrostatic pressure treatment>>

When the decellularized cell aggregate is obtained by the high hydrostatic pressure treatment, hydrostatic pressure of 50 to 1500 MPa is applied to the obtained decellularized cell aggregate in the medium. From the viewpoint of sufficient decellularization, the applied hydrostatic pressure is preferably 50 MPa or more. From the viewpoint of not requiring a pressure vessel that can withstand the pressurization, not requiring much energy, and preventing the cultured cell aggregate from being damaged by ice formation when the medium used for the application is a water-based medium, the applied hydrostatic pressure is preferably 1500 MPa or less. The applied hydrostatic pressure of 80 to 1300MPa is more preferable, 90 to 1200MPa is even more preferable, 95 to 1100MPa is even more preferable, 95 to 700MPa is even more preferable, and 400 to 700MPa is most preferable, from the viewpoint of the decellularization effect, bacteriostatic effect and virus inactivation effect, and ease of pressurization.

The media used for hydrostatic pressure application include water, saline solution, injection water, propylene glycol or its aqueous solution, glycerin or its aqueous solution, sugar aqueous solution, or the like. Buffer solutions include acetate buffer, phosphate buffer, citrate buffer, borate buffer, tartrate buffer, Tris buffer, HEPES buffer, MES buffer, or the like. These media may contain surfactants.

The temperature of the high hydrostatic pressure treatment is not particularly limited as long as ice does not form and heat does not damage the cultured cell aggregate. The temperature is preferably 0 to 45°C, more preferably 4 to 37°C, and most preferably 5 to 35°C, because the decellularization process is smoothly performed and the effect on the cultured cell aggregate is minimal. If the duration of the high hydrostatic pressure treatment is too short, the cells are not sufficiently destroyed, and if the duration of the high hydrostatic pressure treatment is too long, energy is wasted. Therefore, the duration to maintain the desired applied pressure in the high hydrostatic pressure treatment is preferably 1 to 120 minutes, more preferably 5 to 60, and even more preferably 7 to 30.

The cultured cell aggregate treated with high hydrostatic pressure is preferably treated with nucleolytic enzymes. The nucleolytic enzyme removes nucleic acid components from the hydrostatically treated, cultured cell aggregate, and is not particularly limited to, for example, DNase (such as DNase I, DNase II) derived from pancreas, spleen, or E. coli.

The nucleolytic enzyme can be added to the medium (such as water, saline solution, injection solution, or buffer solution) used for the above high hydrostatic pressure treatment, to make the enzyme work. The amount of enzyme to be added depends on the type of enzyme and the definition of the number of units (U), but can be set appropriately by a person skilled in the art. In the case of DNase I, for example, 50~2000 U/mL may be used. The treatment temperature also depends on the nucleolytic enzyme to be used, but for example, may be set at 1°C to 40°C. The treatment time is not particularly limited, but for example, may be set to 1 to 120 hours (preferably 1 to 96 hours, more preferably 1 to 72 hours). In case of low temperatures, the treatment may be long, and in case of high temperatures, the treatment time may be short.

The cultured cell aggregate treated with high hydrostatic pressure is washed with a washing liquid. The washing liquid may be the same as or different from the medium used for the high hydrostatic pressure treatment. The washing liquids preferably contain organic solvents or chelating agents. Organic solvents can improve the removal efficiency of lipids, and chelating agents can inactivate calcium and magnesium ions in the decellularized cell aggregate, thereby preventing calcification when the particulate decellularized cell aggregate of the present invention is applied to diseased areas. As organic solvents, water-soluble organic solvents are preferable because they are effective in removing lipids, and ethanol, isopropanol, acetone, and dimethyl sulfoxide are preferable. As chelating agents, there may be mentioned iminocarboxylic acid chelating agents, such as ethylenediaminetetraacetic acid (EDTA), nitrilotriacetic acid (NTA), diethylenetriamine pentaacetic acid (DTPA), hydroxyethylenediaminetriacetic acid (HEDTA), triethylenetetramine hexaacetic acid (TTHA), 1,3-propanediaminetetraacetic acid (PDTA), and 1,3-diamino-2-hydroxypropane tetraacetic acid (DPTA-OH), hydroxyethyl iminodiacetic acid (HIDA), dihydroxyethylglycine (DHEG), glycol ether diamine tetraacetic acid (GEDTA), dicarboxymethylglutamic acid (CMGA), 3-hydroxy-2, 2'-iminodisuccinic acid (HIDA), dicarboxymethyl aspartate (ASDA) or their salts; hydroxycarboxylic chelating agents, such as citric acid, tartaric acid, malic acid and lactic acid, or their salts. The salts of these chelating agents include sodium or potassium salts.

The washing temperature is not particularly limited as long as there is no heat damage to the decellularized cell aggregate. The washing temperature is preferably 0 to 45°C, more preferably 1 to 40°C, and most preferably 2 to 35°C, since the washing property is good and there is little effect on the decellularized cell aggregate. When washing, the washing liquid may be shaken or stirred as necessary.

By decellularizing the cultured cell aggregate obtained in step (1) by high hydrostatic pressure treatment under the above-mentioned conditions, it is possible to obtain decellularized cell aggregate having 3 to 45 surface pores with a pore diameter of 5 µm or more per 11088 µm² of the aggregate surface, or decellularized cell aggregate containing 10 pg/mg or more of lamin with respect to the decellularized cell aggregate.

### <<Freezing and thawing method>>

When the decellularized cell aggregate is obtained by the freezing and thawing method, the cultured cell aggregate is frozen at a temperature of -80 to -20°C (preferably - 80 to -40°C) for 15 minutes to 48 hours (preferably 20 minutes to 15 hours), and then thawed at a temperature of 20 to 40°C. The above process is repeated once or more than twice (preferably 2 to 5 times). Then, the cell aggregate is preferably treated with nucleolytic enzymes in the same way as the high hydrostatic pressure treatment. The cells in the frozen and thawed cultured cell aggregates are destroyed, and these cells are removed by the washing liquid. The washing method is the same as that in the high hydrostatic pressure treatment.

By decellularizing the cultured cell aggregate obtained in step (1) by freezing and thawing method under the above-mentioned conditions, it is possible to obtain decellularized cell aggregate having 3 to 45 surface pores with a pore diameter of 5 µm or more per 11088 µm² of the spheroid surface, or decellularized cell aggregate containing 10 pg/mg or more of lamin with respect to the decellularized cell aggregate.

### <<Ultrasonic treatment>>

When the decellularized cell aggregate is obtained by the ultrasonic treatment, the cultured cell aggregate is treated with ultrasonic waves (for example, intensity: 10W/cm², frequency: 10 kHz, duration: 2minutes), for example, in a saline solution. Then, the cultured cell aggregate are preferably shaken with a surfactant solution (such as 1 mass% Triton X (polyoxyethylene octylphenyl ether) solution) at 2 to 10°C (preferably 4°C) for 1 to 120 hours (preferably 12 to 120 hours). Then, the cultured cell aggregate is preferably treated with nucleolytic enzymes in the same way as the high hydrostatic pressure treatment. Then, it is preferably washed in the same way as the high hydrostatic pressure treatment.

By decellularizing the cultured cell aggregate obtained in step (1) by ultrasonic treatment under the above-mentioned conditions, it is possible to obtain decellularized cell aggregate having 3 to 45 surface pores with a pore diameter of 5 µm or more per 11088 µm² of the aggregate surface, or decellularized cell aggregate containing 10 pg/mg or more of lamin with respect to the decellularized cell aggregate.

### <<Surfactant treatment>>

When the decellularized cell aggregate is obtained by the surfactant treatment, the cultured cell aggregate is shaken with a surfactant solution (such as RIPA buffer) for 1 to 48 hours (preferably 12 to 36 hours) at 2 to 10°C (preferably 4°C). Then, the cultured cell aggregate is preferably treated with nucleolytic enzymes in the same way as the high hydrostatic pressure treatment. Then, it is preferably washed in the same way as the high hydrostatic pressure treatment.

Surfactant is not limited to, for example, but include RIPA buffer (Nacalai Tesque), sodium dodecyl sulfate (SDS), alkyl sulfonate, alkylsulfate ester salt, polyoxyethylene alkyl alkylsulfate ester salt, α-sulfofatty acid ester salt, polyoxyethylene alkyl ether, polyoxyethylene alkyl phenyl ether (e.g., polyoxyethylene octyl phenyl ether, Nonidet (registered trademark) P-40 (NP-40)), alkyl (poly)glycoside, and alcohol ethoxylate.
Preferably, it is RIPA buffer, Nonidet(registered trademark)P-40 (NP-40), or alcohol ethoxylate. Although not limited thereto, in order to achieve the effects of the present invention, a surfactant with relatively weak cleaning power is preferrable.

The obtained decellularized cell aggregate may be subjected to, but are not limited to, a freeze-dried treatment. Freeze-drying may be omitted depending on the part of the cultured cell aggregate. The obtained decellularized cell aggregate may also be sterilized by gamma irradiation, UV irradiation, or the like.

### <<Area maintenance rate>>

The decellularized cell aggregate obtained by the preparing method of the present invention has a certain area maintenance rate. The area maintenance rate means the percentage of the decellularized cell aggregate decellularized in step (2) relative to the cultured cell aggregate obtained in step (1).

The area maintenance rate is not particularly limited as long as the effect of the present invention is obtained, but the lower limit is 50% or more, 55% or more in one embodiment, 60% or more in one embodiment 65% or more in one embodiment, 70% or more in one embodiment. The upper limit is not particularly limited, but is preferably 99% or less, 95% or less in one embodiment 90% or less in one embodiment. The upper limit and the lower limit can be appropriately combined as the range of the area maintenance rate. In addition, an area maintenance rate of 50 to 99% can be obtained by decellularizing the cultured cell aggregate obtained in step (1) by high hydrostatic pressure treatment, freezing and thawing treatment, or ultrasonic treatment under the above-mentioned conditions.

### <<Surface pore>>

The decellularized cell aggregate obtained by the preparing method of the present invention has 3 to 45 surface pores with a pore diameter of 5 µm or more per 11,088 µm² of the aggregate surface. The number of surface pores per 11,088 µm² of the aggregate surface is not particularly limited as long as it is 3 to 45, but the lower limit is preferably 3 or more in one embodiment, 4 or more in one embodiment, 5 or more in one embodiment, 6 or more in one embodiment, 7 or more in one embodiment, 8 or more in one embodiment, 9 or more in one embodiment, 10 or more in one embodiment, 11 or more in one embodiment, 12 or more in one embodiment, 13 or more in one embodiment, 14 or more in one embodiment, and 15 or more in one embodiment. The upper limit of the number of surface pores is 45 or less, 43 or less in one embodiment, 41 or less in one embodiment, 39 or less in one embodiment, 37 or less in one embodiment, 35 or less in one embodiment, 33 or less in one embodiment, 31 or less in one embodiment, 30 or less in one embodiment, 28 or less in one embodiment, 26 or less in one embodiment, 24 or less in one embodiment, 22 or less in one embodiment, 20 or less in one embodiment, 18 or less in one embodiment. The upper and lower limits can be combined to form a range of the number of surface pores. The survival number of cells can be increased by keeping within the above range. The number of surface pores increases when the intensity (e.g., hydrostatic pressure, time, temperature) of the high hydrostatic pressure treatment is increased, and decreases when the intensity is decreased. Furthermore, the number of surface pores increases when the number of times of freezing and thawing is increased, and decreases when the number of times thereof is decreased. Furthermore, the number of surface pores increases when the intensity (e.g., intensity, frequency, time) of the ultrasonic treatment is increased, and decreases when the intensity thereof is decreased. Therefore, a person skilled in the art can understand from the description of the present specification that decellularized cell aggregates having various numbers of surface pores can be obtained by adjusting the intensity of the decellularization treatment.

### <<Remaining lamin>>

The decellularized cell aggregate obtained by the preparing method of the present invention contains lamin at 10 pg/mg or more with respect to the decellularized cell aggregate. The lamin content is not particularly limited as long as it is 10 pg/mg or more, but the lower limit is preferably 15 pg/mg or more in one embodiment, 18 pg/mg or more in one embodiment, 20 pg/mg or more in one embodiment, 25 pg/mg or more in one embodiment, 30 pg/mg or more in one embodiment, 35 pg/mg or more in one embodiment, 40 pg/mg or more in one embodiment, 45 pg/mg or more in one embodiment, 50 pg/mg or more in one embodiment, 60 pg/mg or more in one embodiment, 80 pg/mg or more in one embodiment, 100 pg/mg or more in one embodiment, 110 pg/mg or more in one embodiment, 120 pg/mg or more in one embodiment, 130 pg/mg or more in one embodiment, 135 pg/mg or more in one embodiment. The upper limit is not particularly limited, but it is preferably 5000 pg/mg or less in one embodiment, 4000 pg/mg or less in one embodiment, 3000 pg/mg or less in one embodiment, 2000 pg/mg or less in one embodiment, 1000 pg/mg or less in one embodiment, 900 pg/mg or less in one embodiment, 800 pg/mg or less in one embodiment, 700 pg/mg or less in one embodiment, 600 pg/mg or less in one embodiment, 500 pg/mg or less in one embodiment, 450 pg/mg or less in one embodiment, 400 pg/mg or less in one embodiment, 350 pg/mg or less in one embodiment, 300 pg/mg or less in one embodiment, 250 pg/mg or less in one embodiment, 200 pg/mg or less in one embodiment, 150 pg/mg or less in one embodiment. The upper and lower limits can be appropriately combined to define a range of lamin content. An excellent cell viability is exhibited by keeping the lamin content within the above range. The lamin content decreases when the intensity (e.g., hydrostatic pressure, time, temperature) of the high hydrostatic pressure treatment is increased, and increases when the intensity is increased. Furthermore, the lamin content decreases when the number of times of freezing and thawing is increased, and increases when the number of times thereof is decreased. Furthermore, the lamin content decreases when the intensity (e.g., intensity, frequency, time) of the ultrasonic treatment is increased, and increases when the intensity thereof is decreased. Therefore, a person skilled in the art can understand from the description of the present specification that decellularized cell aggregates having various lamin content can be obtained by adjusting the intensity of the decellularization treatment.

The size of the decellularized cell aggregate is not particularly limited, but in the case of a decellularized spheroid, the lower limit is preferably 80 µm or more, preferably 90 µm or more, preferably 95 µm or more, and preferably 100 µm or more. The upper limit is preferably 600 µm or less, preferably 500 µm or less, and preferably 300 µm or less. In the case of a decellularized cell block, the lower limit is preferably 90 µm or more, preferably 100 µm or more, preferably 150 µm or more, and preferably 200 µm or more. The upper limit is preferably 2100 µm or less, preferably 2000 µm or less, preferably 1700 µm or less, preferably 1400 µm or less, preferably 1200 µm or less, and preferably 1000 µm or less. The upper and lower limits can be combined to define the size range of the decellularized cell aggregate. The survival number of cells can be increased by keeping within the above range.

In the present disclosure, the following enbodiments are included.
[1] A decellularized cell aggregate, wherein a cultured cell aggregate is decellularized, and surface pores with pore diameter of 5µm or more are 3 to 45 per 11088µm² of aggregate surface.
[2] A decellularized cell aggregate, wherein a cultured cell aggregate is decellularized, and lamin of 10pg/mg or more is contained with respect to the decellularized cell aggregate.
[3] A decellularized cell aggregate, wherein a cultured cell aggregate is decellularized, surface pores with pore diameter of 5µm or more are 3 to 45 per 11088µm² of aggregate surface, and lamin of 10pg/mg or more is contained with respect to the decellularized cell aggregate.
[4] The decellularized cell aggregate of any one of the items [1] to [3], wherein cells of the cultured cellular aggregate are immortalized cells.
[5] The decellularized cell aggregate of any one of the items [1] to [4], wherein cells of the cultured cellular aggregate are a mixture of two or more kinds of cells.
[6] The decellularized cell aggregate of any one of the items [1] to [5], wherein a DNA content in dry mass is 1.000 % by weight or less with respect to the decellularized cell aggregate.
[7] A cell culture substrate comprising the decellularized cell aggregate of any one of the items [1] to [6].
[8] A graft for living body comprising the decellularized cell aggregate of any one of the items [1] to [6].
[9] A composition comprising the decellularized cell aggregate of any one of the items [1] to [6], and cells.
[10] A method for preparing a decellularized cell aggregate comprising a formation step of cultured cell aggregate wherein the cultured cell aggregate is formed by culturing cells, and a decellularization step wherein the cultured cell aggregate is decellularized, wherein surface pores with pore diameter of 5µm or more are 3 to 45 per 11088µm² of aggregate surface.
[11] a method for preparing a decellularized cell aggregate comprising a formation step of cultured cell aggregate wherein the cultured cell aggregate is formed by culturing cells, and a decellularization step wherein the cultured cell aggregate is decellularized, wherein lamin of 10pg/mg or more is contained with respect to the decellularized cell aggregate.
[12] the method for preparing a decellularized cell aggregate of the item [10] or [11], wherein the cells are immortalized cells.
[13] the method for preparing a decellularized cell aggregate of any one of the items [10] to [12], wherein the culture of cells is a culture of two or more kinds of cells.
[14] The method for preparing a decellularized cell aggregate of any one of the items [10] to [13], wherein the decellularization is performed by high hydrostatic pressure treatment.

The present invention is not limited to the above-mentioned embodiment. The above embodiment is merely an example, and anything that has substantially the same configuration as the technical idea described in the claims of the present invention and exhibits similar effects is comprised within the technical scope of the present invention.

### EXAMPLES

The present invention will now be further illustrated by, but is by no means limited to, the following Examples.

### <<Example 1>>

In this example, decellularized cell aggregates (decellularized spheroids) were prepared using fibroblasts.

### (Preparation of cultured cell aggregates (spheroids))

NHDF-Neo-dermal fibroblasts (CC-2509, Lonza) were cultured in a T-175 flask (vent cap, 175cm², Violamo). The cells were detached at about 90-100% confluency and cell suspension (20 mL) was prepared (about 2.0×10⁷ cells), using FKCM medium (serum-free medium for fibroblasts, Fukoku). A dimple plate was placed in a 10 cm dish, and the above cell suspension was poured evenly into the dimple plate and cultured in a 37°C, 5% CO₂ incubator (IP400, Yamato Scientific Co., Ltd). After culturing overnight or more, the spheroids (aggregations of cells) in the dimple plate were observed under an inverted microscope (CKX31, Olympus). The spheroids were pipetted to remove them from depressions of the dimple plate and transferred to a 10 cm dish. The 10 cm dish was rotated to collect the spheroids in the center, and the spheroids were obtained.

### (Decellularization treatment of spheroids)

The spheroids and FKCM medium were placed in a nylon poly vacuum bag (Kyo Bijin, Kurilon Kasei Co., Ltd.) and it was double-sealed on all four sides with a sealer. The high hydrostatic pressure treatment was carried out using the FKCM medium as a medium, at 600 MPa for 10 minutes using a high-pressure apparatus for research and development (Dr. CHEF, Kobe Steel, Ltd.). The high hydrostatic pressure treatment was carried out at a temperature range of a minimum temperature of 23.3°C and a maximum temperature of 35.9°C. The high hydrostatic pressure-treated spheroids were recovered from the nylon poly vacuum bag, transferred to a centrifuge tube, and washed with water for injection. Then, the high hydrostatic pressure-treated spheroids were immersed in a solution of DNaseI (800 U/mL), nucleic acid degrading enzyme, and washed by shaking at 4°C for more than 18 hours. Further, the spheroids were washed three times with water for injection, followed by freeze-drying to obtain decellularized spheroids (decellularized cell aggregates).

### <<Example 2>>

In this example, decellularized spheroids were prepared by freezing and thawing treatment.

The spheroids obtained in Example 1 were washed with water for injection, suspended in phosphate buffer (PBS, 0.01M, pH 7.4), frozen on dry ice or in a freezer, and stored at -80°C for 30 minutes or more, and then thawed in a water bath at 37°C. The spheroids were subjected to this freeze-thawing process three times, immersed in a solution of DNase I (800U/mL), nucleic acid degrading enzyme, and washed by shaking at 4°C for 18 hours or more. The spheroids were further washed three times with water for injection and freeze-dried to obtain decellularized spheroids.

### <<Example 3>>

In this example, decellularized cell aggregates (decellularized spheroids) were prepared using immortalized cells.

### (Preparation of cultured cell aggregates (spheroids))

The procedure described in Example 1 was repeated, except that OUMS-36T-1 immortalized fibroblasts (JCRB 1006.1, JCRB Cell Bank) were used instead of NHDF-Neo-dermal fibroblasts, to obtain the spheroids.

### (Decellularization treatment of spheroids)

Decellularized spheroids (decellularized cell aggregates) were obtained using the same procedure as in Example 1.

### <<Example 4>>

In this example, decellularized cell aggregates (decellularized spheroids) were prepared using fibroblasts and endothelial cells.

### (Preparation of cultured cell aggregates (spheroids))

NHDF-Neo-dermal fibroblasts (CC-2509, Lonza) and HUEhT-1 immortalized vascular endothelial cells (JCRB1458, JCRB Cell Bank) were cultured in a T-175 flask (vented cap, 175 cm₂, Bioramo), respectively.

When the cells reached a confluency rate of approximately 90 to 100%, they were detached, and a cell suspension (20 mL) was prepared using a medium that was a mixture of equal amounts of FKCM medium (serum-free medium for fibroblasts, Fukoku) and CS-C medium (CELL SYSTEMS CORPORATION) so that the number of NHDF cells:HUEhT-1 cells was 9:1(about 1.7×10⁷cells). The above cell suspension was evenly distributed on the dimple plate and co-cultured in a 37°C, 5% CO₂ incubator (IP400, Yamato Scientific). After culturing overnight or more, the spheroids (aggregations of cells) in the dimple plate were observed under an inverted microscope (CKX31, Olympus). The spheroids were pipetted to remove them from depressions of the dimple plate and transferred to a 10 cm dish. The 10 cm dish was rotated to collect the spheroids in the center, and the spheroids were obtained.

### (Decellularization treatment of spheroids)

Decellularized spheroids (decellularized cell aggregates) were obtained using the same procedure as in Example 1.

### <<Example 5>>

In this example, decellularized cell aggregates (decellularized spheroids) were prepared using fibroblasts and vascular endothelial cells.

### (Preparation of cultured cell aggregates (spheroids))

NHDF-Neo-dermal fibroblasts (CC-2509, Lonza) and HUEhT-1 vascular endothelial cells (JCRB1458, JCRB Cell Bank) were cultured in a T-175 flask (vented cap, 175 cm₂, Bioramo), respectively.

When the cells reached a confluency rate of approximately 90 to 100%, they were detached, and a cell suspension (20 mL) was prepared using a medium that was a mixture of equal amounts of FKCM medium (serum-free medium for fibroblasts, Fukoku) and CS-C medium (CELL SYSTEMS CORPORATION) so that the number of NHDF cells:HUEhT-1 cells was 7:3(about 1.7×10⁷cells). The above cell suspension was evenly distributed on the dimple plate and co-cultured in a 37°C, 5% CO₂ incubator (IP400, Yamato Scientific). After culturing overnight or more, the spheroids (aggregations of cells) in the dimple plate were observed under an inverted microscope (CKX31, Olympus). The spheroids were pipetted to remove them from depressions of the dimple plate and transferred to a 10 cm dish. The 10 cm dish was rotated to collect the spheroids in the center, and the spheroids were obtained.

### (Decellularization treatment of spheroids)

Decellularized spheroids (decellularized cell aggregates) were obtained using the same procedure as in Example 1.

### <<Example 6>>

In this example, the spheroids were decellularized using the same procedure as in Example 1, and then irradiated with gamma rays (25 kGy) to prepared decellularized cell aggregates (decellularized spheroids).

### (Preparation of cultured cell aggregates (spheroids))

Spheroids were obtained using the same procedure as in Example 1

### (Decellularization treatment of spheroids)

The same procedure as in Example 1 was followed up to freeze-dried treatment, and then the cells were irradiated with gamma rays (25 kGy) to obtain decellularized spheroids (decellularized cell aggregates).

### <<Example 7>>

In this example, the decellularization treatment was performed by surfactant treatment. The spheroids obtained in Example 1 were decellularized by the following surfactant treatment.

The spheroids obtained in Example 1 were washed with water for injection. Then, the spheroids were treated with a protein extraction buffer containing a surfactant (RIPA buffer, Nacalai Tesque) at 4°C for 24 hours or more.
The treated spheroids were then immersed in a solution of DNase I (800 U/mL) , nucleic acid degrading enzyme and washed by shaking at 4°C for 18 hours or more. The spheroids were further washed three times with water for injection and then freeze-dried to obtain decellularized spheroids (decellularized cell aggregates).

### <<Example 8>>

In this example, a decellularized cell block was prepared as a decellularized cell aggregate.

### (Preparation of cell block as cultured cell aggregate)

The spheroids obtained in Example 1 were placed in a net mold (Net Mold Starter Kit V6, Tissue By Net). The net mold was transferred to a 120 mL sterile 4-ounce container (Thermo Fisher Scientific) containing a mixed medium (40 mL) of FKCM medium and DMEM medium, and placed on a shaker (OS-762, Optima) in a 5% CO₂ incubator at 37°C, and a culture was started under shaking (45 rpm). Half of the medium was replaced once or twice a week, and the culture was continued. After about 3 weeks of culture, the net mold was removed, and the cell block was obtained as a cultured cell aggregate.

### (Decellularization treatment of cell block)

The cell block removed from the net mold and a mixed medium were placed in a nylon poly vacuum bag (Kyo Bijin, Kurilon Kasei Co., Ltd.) and it was double-sealed on all four sides with a sealer. The high hydrostatic pressure treatment was carried out using the FKCM medium as a medium, at 600 MPa for 10 minutes using a high-pressure apparatus for research and development (Dr. CHEF, Kobe Steel, Ltd.). The high hydrostatic pressure treatment was carried out at a temperature range of a minimum temperature of 23.3°C and a maximum temperature of 35.9°C. The cultured cell structure treated with high hydrostatic pressure was removed from the nylon poly vacuum bag, immersed in water for injection, and washed by shaking at 4°C for 5 minutes or more. Then, the cultured cell structure treated with high hydrostatic pressure were immersed in a solution of DNase I (800 U/mL), nucleic acid degrading enzyme, and washed by shaking at 4°C for more than 18 hours. Further, the cultured cell structure was immersed in water for injection, and washed three times by shaking at 4°C for 5 minutes or more, to obtain the decellularized cell block as the decellularized cell aggregate.

### <<Comparative Example 1>>

In this comparative example, decellularization treatment was performed by surfactant treatment. The spheroids obtained in Example 1 were decellularized by the following surfactant treatment.

The spheroids obtained in Example 1 were washed with water for injection. The spheroids were then treated with 0.25% by mass sodium dodecyl sulfate solution (10 mM
Tris, pH 8.0) at 4°C for 24 hours or more. Subsequently, the treated spheroids were treated with 0.5% by mass Triton-X (polyoxyethylene octylphenyl ether) solution (10 mM Tris, pH 8.0) at 4°C for 24 hours or more. Further, the spheroids were further washed once with water for injection and then freeze-dried to obtain decellularized spheroids.

### <<Comparative Example 2>>

In Comparative Example 2, a gelatin fiber substrate for cell culture was used.

### <<Calculation of decellularized DNA ratio >>

The mass of the decellularized cell aggregates of Examples 1 to 8 and Comparative Example 1 was measured, and these samples were dissolved by immersion in a protease solution, and then treated with phenol/chloroform to remove proteins and DNA is recovered. The recovered DNA is fluorescently stained with PicoGreen (Life Technologies) and the DNA is quantified by measuring the fluorescence intensity, and the DNA content (mass) per sample mass is calculated from the sample mass and DNA amount. For quantification of DNA, a calibration curve prepared using the standard DNA provided with Picogreen was used.
(Decellularized DNA ratio) = (DNA content of dried specimen of decellularized spheroid or decellularized cell block (decellularized cell spheroid))/(mass of dried specimen of decellularized spheroid or decellularized cell block (decellularized cell aggregate)) × 100

**[Table 1]**

| | DNA content(ng) (A) | Mass of specimen(mg) (B) | Decellularized DNA ratio (A/B x 100; Mass %) |
|---|---|---|---|
| Example 3 | 5166 | 2.6 | 0.199 |
| Example 4 | 4760 | 1.3 | 0.340 |
| Example 5 | 3280 | 2.2 | 0.149 |
| Example 6 | 6475 | 2.5 | 0.259 |
| Example 1 | 4052 | 2.3 | 0.176 |
| Example 8 | 1182 | 2.4 | 0.049 |
| Example 7 | 14322 | 2.1 | 0.682 |
| Example 2 | 4580 | 2.0 | 0.229 |
| Comparative Example 1 | 47848 | 2.4 | 1.990 |

The results are shown in Table 1. It was found that decellularization was more successful in the Example than in the Comparative Example. This demonstrates that the Example can be used as a decellularized graft with less rejection.

### <<Calculation of area maintenance rate>>

The spheroids or cell blocks (before decellularization treatment) and the decellularized spheroids or decellularized cell blocks of Examples 1 to 8 and Comparative Example 1 were observed under a microscope, photographed at 4x magnification, and the areas were quantified using image processing software (WinROOF2013).

The area maintenance rate was calculated from the area of the spheroids or cell blocks, and the area of the decellularized spheroids or decellularized cell blocks of the Examples and Comparative Examples. The area of the spheroids or cell blocks before processing is set to 100%. Area maintenance rate (%) = (area of decellularized spheroid or decellularized cell block / area of spheroid or cell block) x 100

The results of calculating the area maintenance rate are shown in Figure 1. As for the area maintenance rate, it was found that the area after decellularization with respect to the area before decellularization of the Examples was maintained compared to the Comparative Example. The area in example 1 was particularly maintained before and after decellularization. In particular, further, the areas in Examples 3, 4, 5, and 8 were well maintained before and after decellularization.

For Example 3, the following is presumed. By using immortalized cells to prepare decellularized cell aggregates, they contain a lot of lamin B1 described later, and the adhesion between cells becomes stronger. In addition, by using cells with the same genotype and phenotype, the size of the cells becomes uniform, making it easier for the cells to adhere to each other. For Examples 4 and 5, it is presumed that the decellularized cell aggregates were prepared by co-culturing cells, and whereby they contain a lot of lamin B1 described later, and the adhesion between cells becomes stronger. For Example 8, it is presumed that the decellularized cell block was prepared as the decellularized cell aggregate, and whereby it contains a lot of lamin B1 described later by long-term culture, and the adhesion between cells becomes stronger. Example 6 shows that the decellularized cell aggregates of Example 1 are able to maintain their area compared to the comparative example, even when irradiated with gamma rays. This shows that the examples can be used better as scaffolding materials and grafts than the comparative example, and it is presumed that they promote cell activity, which will be described later.

### <<Measurement of surface pores>>

The obtained decellularized spheroids, decellularized cell blocks, or Comparative Example 2 were observed with a scanning electron microscope (SEM) and photographed at 1000x magnification. Using image processing software (WinROOF2013), the surface pores of the decellularized spheroids and decellularized cell blocks of Examples 1-8 and Comparative Example 1, or Comparative Example 2 were measured. The measurement area was set to 88µm× 126µm, and the number of surface pores with a diameter of 5µm or more was counted. The number of surface pores that were entirely contained within the measurement area were counted.

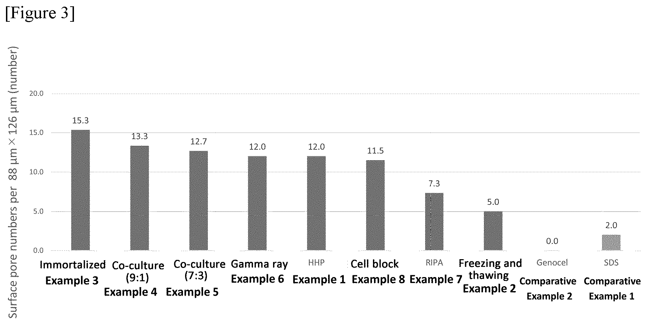

The surface appearance of the decellularized spheroid, decellularized cell block, or Comparative Example 2 are shown in Figure 2. The result of measuring the number of surface pores of the decellularized spheroid, decellularized cell block, or Comparative Example 2 is shown in Figure 3. In the Examples, more surface pores existed than the Comparative Examples, and Examples 1 and 8 had particularly many surface pores. Moreover, Examples 3, 4, and 5 had even more surface pores.

For Example 3, the following is presumed. By preparing decellularized cell aggregates using immortalized cells, it contains a large amount of lamin B1 described later. In addition, the size of the cells is uniform by using cells having the same genotype and phenotype, and whereby a densely packed cultured cell aggregate was prepared and it was decellularized. For Examples 4 and 5, the following is presumed. The decellularized cell aggregates were prepared by co-culturing cells, and whereby they contain a lot of lamin B1 described later, and a densely packed cultured cell aggregate was prepared and this was decellularized. From Example 6, it was found that the decellularized cell aggregate of Example 1 was able to maintain the number of surface pores compared to the Comparative Example, even when irradiated with gamma rays. From these results, it is presumed that the Example promotes cell activity, which will be described later, compared to the Comparative Example, and it is considered that the Example can be used favorably as a scaffold material or a graft.

### <<Measurement of cell viability>>

The decellularized spheroids, decellularized cell block, or Comparative Example 2 of Examples and comparative Examples were suspended in DMEM medium to prepare a 1.0 mg/mL suspension, and 100 µL of the suspension (0.1 mg/well) was added to a 96-well U-bottom plate (PrimeSurface plate 96U, Sumitomo Bakelite). 50 µL of a cell suspension of NHDF-Neo-dermal fibroblasts (CC-2509, Lonza) at 2.0 × 10⁵ cells/mL was seeded into the above added wells (1.0 × 10⁴ cells/well), and the cells were cultured at 37 °C in a 5% CO₂ incubator for 10 days while changing the medium every two days. After the culture, a cell viability assay was performed using CellTiter-Glo 3D Cell Viability Assay (Promega), and the cell viability was measured by measuring the amount of cellular ATP in terms of amount of luminescence (Relative light unit; RLU).

The results of cell viability measurement are shown in FIG. 4. It was found that the Examples had higher cell viability and better nutrient supply effect to cells than the Comparative Example. This showed that cell proliferation was promoted by seeding cells on decellularized spheroids, decellularized cell block of Examples. In Examples 1 and 8, cell viability was particularly high. Also, in Examples 3, 4, and 5, cell viability was further higher. For Example 3, it is presumed that by preparing the decellularized cell aggregate using immortalized cells, the area maintenance rate and the number of surface pores can be effectively set to a predetermined value. For Examples 4 and 5, it is presumed that the decellularized cell aggregates were prepared by co-culturing cells, and whereby the area maintenance rate and the number of surface pores can be effectively set to a predetermined value. From Example 6, it was found that the decellularized cell aggregate of Example 1 was able to maintain the cell viability compared to the Comparative Example, even when irradiated with gamma rays. It was also confirmed that spheroids could be successfully prepared again by culturing the decellularized spheroids of the Examples with cells. For the Examples, it was found that the decellularized grafts can be prepared using a simple procedure and can be used as a decellularized grafts that shows good tissue regeneration.

### <<Preparation of paraffin-embedded tissue specimens and section sample for staining>>

As pretreatment, the decellularized spheroid, and decellularized cell block obtained in Examples 1 to 8, or Comparative Example 2 were gelled with IPGell (GenoStuff) and immersed in 4 mass% PFA (4 % by mass paraformaldehyde phosphate buffer, for tissue fixation by FUJIFILM Wako Pure Chemicals Corporation) for at least 24 hours. Then, the gels were immersed in phosphate buffer solution (PBS, 0.01M, pH 7.4) and washed at 4°C for 30 minutes or more with shaking. Subsequently, the gels were immersed in 70 % by mass ethanol and washed at 4°C for 30 minutes or more with shaking, and then immersed again in 70% by mass ethanol to prepare a sample for preparing paraffin-embedded tissue specimens.

Paraffin-embedded tissue specimens were prepared from the above samples according to the following procedure, and section samples for staining were prepared for staining (see below).
Tissues were rinsed with tap water for 30 to 40 minutes to remove formaldehyde. Samples were dehydrated in an ethanol bath using a paraffin embedding apparatus (CT-Pro20, Genostaff) in the following order:
70 mass% ethanol for 20 minutes (×1)
95 mass% ethanol for 20 minutes (×2)
100 mass% ethanol for 20 minutes (×2)

Further, the samples were washed twice for 20 minutes each, in the low-toxicity solvent G-Nox (GenoStaff), an alternative to xylene. The samples were incubated twice in a paraffin bath at 65°C for 30 minutes each. A melted paraffin was poured into a mold, the sample was placed in the mold, and allowed to cool for 15 to 20 minutes to prepare a paraffin-embedded tissue specimen. Then, the paraffin-embedded tissue specimens were cut into 5-µm-thick serial sections on a microtome and floated in a 37°C water bath filled with deionized water. The sections were floated one by one in distilled water on a glass slide, warmed on a stretching table (30-40°C), and dried in a constant incubator (37°C) for 1 to 2 nights to make section samples for staining.

### <<LaminB1 detection (Immunohistochemical staining)>>

The decellularized spheroids and decellularized cell blocks obtained in Examples 1-8 or Comparative Example 1, or Comparative Example 2 were immunohistochemically stained and lamin stained according to the following procedure.

First, the section samples for staining were deparaffinized and hydrophilized.
Then, after washing three times with phosphate buffer (PBS, 0.01M, pH 7.4), the samples were immersed in EDTA buffer (pH 9.0): G-Activate (antigen activation buffer, pH 9.0) and heat-treated to activate the antigens.

Next, the samples were washed three times with phosphate buffer (PBS, 0.01M, pH 7.4), treated with 0.3% hydrogen peroxide/methanol at room temperature for 30 minutes, and washed three times with Tris-buffered saline. Then, the samples were treated with G-Block (GenoStaff) at room temperature for 10 minutes, and then blocked by treating with Avidin/Biotin Blocking kit (VECTOR LABORATORIES).

Next, Lamin B1 rabbit monoclonal antibody (17416, Cell Signaling) was used as a primary antibody, to carry out an antibody reaction overnight at 4°C using. The sections were then washed twice with Tween 20-containing Tris-buffered saline, followed by washing with Tris-buffered saline.

Next, Anti-Rabbit Ig Biotin (E0432, Dako) was used as a secondary antibody, to carry out an antibody reaction for 30 minutes at room temperature. The sections were then washed twice with Tween 20-containing Tris-buffered saline, followed by washing with Tris-buffered saline.

The sections were then treated with peroxidase-labeled streptavidin (Nichirei) for 5 minutes at room temperature. Then, the sections were washed twice with Tween 20-containing Tris-buffered saline, followed by washing with Tris-buffered saline.

The sections were then treated with DAB (3,3'-diaminobenzidine tetrahydrochloride)/hydrogen peroxide to develop color. The cell nuclei were counterstained with Mayer's hematoxylin (Muto Pure Chemicals Co., LTD), as a marker for observation.

Subsequently, the sections were dehydrated and cleared, and then mounted with mounting medium (xylene-based mounting material: Marinol, Muto Pure Chemicals Co., LTD).

The results of immunohistochemical staining are shown in Figure 5. The left photograph of each example (comparative example) shows lamin B1 staining, and the right photograph shows the negative control (stained with rabbit Ig). In the examples, areas stained darker in the left photograph compared to the right photograph were confirmed, and lamin B1 was detected. In contrast, in the comparative examples, there was no difference in staining between the left and right photograps, and lamin B1 was not detected.

### <<Lamin B1 quantification (Enzyme-linked immunosorbent assay)>>

The lamin B1 content of the decellularized spheroids and decellularized cell blocks of the Examples and Comparative Examples, or Comparative Example 2 were quantified by enzyme-linked immunosorbent assay (ELISA).

The Cell Extraction Buffer PTR included with the ELISA kit (Human Lamin B1 ELISA Kit, ab252351, abcam) was added to the decellularized spheroids and decellularized cell blocks of the Examples and Comparative Examples, or Comparative Example 2, and the mixture was homogenized using a disposable homogenizer (BioMasherII, Nippi). After incubation on ice for 20 minutes, the mixture was centrifuged at 18,000Xg for 20 minutes at 4°C, and the supernatant was collected and used as the extract of the decellularized spheroids of the Examples and Comparative Examples. The lamin B1 content of the extract was quantified according to the protocol of the ELISA kit manufacturer. The content of lamin B1 per mass of sample was calculated.

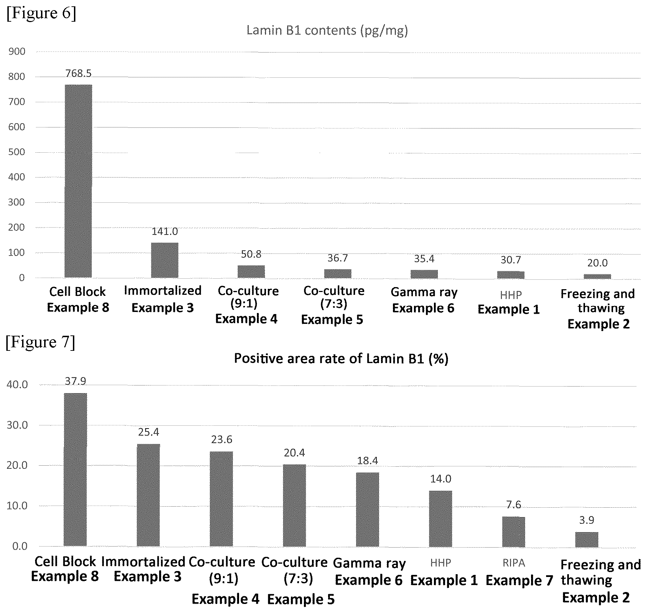

The results of quantifying lamin B1 by enzyme-linked immunosorbent assay are shown in Figure 6. In the Examples, it was found that a large amount of lamin B1 were contained. In Example 1, value of lamin B1 was especially high. Further, in Examples 3, 4, 5, and 8, quantitative values of lamin B1 were even higher. Example 6 showed that the decellularized cell aggregate of Example 1 were able to maintain the quantitative value of lamin B1 compared to the Comparative Example, even when irradiated with gamma rays. The Comparative Example did not contain lamin B1. This demonstrated that the Examples promoted the proliferation of seeded cells.

### <<Calculation of positive area>>

The immunohistochemically stained sections obtained above were observed under a microscope, the sections were photographed at 40x magnification, and the positive area for the examples and comparative examples was calculated using image processing software (ImageJ). The images of the stained sections were imported, and the area of the entire stained section and the area of the lamin B1-positive staining area were measured, respectively.

First, the positive area rate for the examples and comparative examples was calculated according to the following formula. As the measurement area, a square area (30 pixels x 30 pixels) was set within a range of 100 µm in the depth direction from the cell seeding surface, and the average positive area rate for three points was calculated. Positive area rate (%) = Area of lamin B 1-positive staining area / Area of entire stained section (measurement area) x 100

The results of calculating the positive area of immunohistochemical staining are shown in Figure 7. In Examples, it was found that a large amount of lamin B1 was contained. It was found that Example 1 contained particularly large amounts of lamin B1. Further, it was found that Examples 3, 4, 5, and 8 contained even larger amounts of lamin B1. From Example 6, it was found that the decellularized cell aggregate of Example 1 were able to maintain lamin B1 compared to the Comparative Example, even when irradiated with gamma rays. The Comparative Example did not contain lamin B1. This demonstrated that the Examples promote the proliferation of seeded cells.

### INDUSTRIAL APPLICABILITY

The decellularized cell aggregate of the present invention can be used as a substrate for cell culture (scaffold material) and as a graft for a living body.

### SEQUENCE LISTING

```
 <?xml version="1.0" encoding="UTF-8"?>
 <!DOCTYPE ST26SequenceListing PUBLIC "-//WIPO//DTD Sequence Listing 1.3//EN"
 "ST26SequenceListing_V1_3.dtd">
 <ST26SequenceListing dtdVersion="V1_3" fileName="ADK-098.xml"
 softwareName="WIPO Sequence" softwareVersion="2.3.0" productionDate="2023-11-
 29">
         <ApplicantFileReference>ADK-098</ApplicantFileReference>
         <EarliestPriorityApplicationIdentification>
                  <IPOfficeCode> JP</IPOfficeCode>
                  <ApplicationNumberText>2022-195029</ApplicationNumberText>
                  <FilingDate>2022-12-06</FilingDate>
         </EarliestPriorityApplicationIdentification>
         <ApplicantName languageCode="en">ADEKA
 CORPORATION</ApplicantName>
         <InventionTitle languageCode="en">Decellularized cell spheroid and method
         for producing the same</InventionTitle>
         <SequenceTotalQuantity> 1 </SequenceTotalQuantity>
         <SequenceData sequenceIDNumber="1">
                  <INSDSeq>
                            <INSDSeq_length>586</INSDSeq_length>
                            <INSDSeq_moltype>AA</INSDSeq_moltype>
                            <INSDSeq_division>PAT</INSDSeq_division>
                            <INSDSeq_feature-table>
                                     <INSDFeature>
         <INSDFeature_key>source</INSDFeature_key>
         <INSDFeature_location>1..586</INSDFeature_location>
                                              <INSDFeature_quals>
                                                       <INSDQualifier>
         <INSDQualifier_name>mol_type</INSDQualifier_name>
         <INSDQualifier_value>protein</INSDQualifier_value>
                                                       </INSDQualifier>
                                                       <INSDQualifier id="q2">
         <INSDQualifier_name>organism</INSDQualifier_name>
         <INSDQualifier_value>Homo sapiens</INSDQualifier_value>
                                                       </INSDQualifier>
                                              </INSDFeature_quals>
                                     </INSDFeature>
                            </INSDSeq_feature-table>
         <INSDSeq_sequence>MATATPVPPRMGSRAGGPTTPLSPTRLSRLQEKEEL
         RELNDRLAVYIDKVRSLETENSALQLQVTEREEVRGRELTGLKALYETELADARR
 ALDDTARERAKLQIELGKCKAEHDQLLLNYAKKESDLNGAQIKLREYEAALNSK
 DAALATALGDKKSLEGDLEDLKDQIAQLEASLAAAKKQLADETLLKVDLENRCQ
 SLTEDLEFRKSMYEEEINETRRKHETRLVEVDSGRQIEYEYKLAQALHEMREQHD
 AQVRLYKEELEQTYHAKLENARLSSEMNTSTVNSAREELMESRMRIESLSSQLSN
 LQKESRACLERIQELEDLLAKEKDNSRRMLTDKEREMAEIRDQMQQQLNDYEQL
 LDVKLALDMEISAYRKLLEGEEERLKLSPSPSSRVTVSRASSSRSVRTTRGKRKRV
 DVEESEASSSVSISHSASATGNVCIEEIDVDGKFIRLKNTSEQDQPMGGWEMIRKIG
 DTSVSYKYTSRYVLKAGQTVTIWAANAGVTASPPTDLIWKNQNSWGTGEDVKVI
 LKNSQGEEVAQRSTVFKTTIPEEEEEEEEAAGVVVEEELFHQQGTPRASNRSCAIM
 </INSDSeq_sequence>
                  </INSDSeq>
         </SequenceData>
 </ST26SequenceListing>
```

## Claims

1. A decellularized cell aggregate, wherein a cultured cell aggregate is decellularized, and surface pores with pore diameter of 5µm or more are 3 to 45 per 11088µm² of aggregate surface.

2. A decellularized cell aggregate, wherein a cultured cell aggregate is decellularized, and lamin of 10pg/mg or more is contained with respect to the decellularized cell aggregate.

3. A decellularized cell aggregate, wherein a cultured cell aggregate is decellularized, surface pores with pore diameter of 5µm or more are 3 to 45 per 11088µm² of aggregate surface, and lamin of 10pg/mg or more is contained with respect to the decellularized cell aggregate.

4. The decellularized cell aggregate according to any one of claims 1 to 3, wherein cells of the cultured cell aggregate are immortalized cells.

5. The decellularized cell aggregate according to any one of claims 1 to 3, wherein cells of the cultured cell aggregate are a mixture of two or more kinds of cells.

6. The decellularized cell aggregate according to any one of claims 1 to 3, wherein a DNA content in dry mass is 1.000 % by weight or less with respect to the decellularized cell aggregate.

7. A cell culture substrate comprising the decellularized cell aggregate according to any one of claims 1 to 3.

8. A graft for living body comprising the decellularized cell aggregate according to any one of claims 1 to 3.

9. A composition comprising the decellularized cell aggregate according to any one of claims 1 to 3, and cells.

10. A method for preparing a decellularized cell aggregate comprising
a formation step of cultured cell aggregate wherein the cultured cell aggregate is formed by culturing cells, and
a decellularization step wherein the cultured cell aggregate is decellularized,
wherein surface pores with pore diameter of 5µm or more are 3 to 45 per 11088µm² of aggregate surface.

11. A method for preparing a decellularized cell aggregate comprising
a formation step of cultured cell aggregate wherein the cultured cell aggregate is formed by culturing cells, and
a decellularization step wherein the cultured cell aggregate is decellularized,
wherein lamin of 10pg/mg or more is contained with respect to the decellularized cell aggregate.

12. The method for preparing a decellularized cell aggregate according to claim 10 or 11, wherein the cells are immortalized cells.

13. The method for preparing a decellularized cell aggregate according to claim 10 or 11, wherein the culture of cells is a culture of two or more kinds of cells.

14. The method for preparing a decellularized cell aggregate according to claim 10 or 11, wherein the decellularization is performed by high hydrostatic pressure treatment.
